# EUROPEAN PATENT APPLICATION

(11) **EP 4 810 010 A1**
(43) Date of publication of application: **23.09.2026**
(21) Application number: 24891235.4
(22) Date of filing: 31.10.2024
(51) Int. Cl.: A61K 8/46, A61K 8/92, A61Q 5/02

(54) **HAIR WASHING AGENT COMPOSITION**

(30) Priority: 14.11.2023 JP 2023193977
(71) Applicant: Kao Corporation, Tokyo 103-8210 (JP)
(72) Inventor: TORII, Shusaku, Tokyo 103-8210 (JP)
(74) Representative: Hoffmann Eitle
(86) International application number: PCT/JP2024/038869
(87) International publication number: WO 2025/105194

(57) **Abstract**

The present invention relates to a hair cleansing composition which uses a glycolipid type biosurfactant, exerts high performance when applied to hair, and also has excellent low-temperature stability. That is, the present invention is a hair cleansing composition containing the following components (A) and (B): (A) a glycolipid type biosurfactant and (B) an internal olefin sulfonic acid having 16 carbon atoms or a salt, prepared by sulfonation of a raw material olefin having 16 carbon atoms and having an average double bond position of 3.9-position or more and 4.4-position or less.

## Description

### Field of the Invention

The present invention relates to a hair cleansing composition.

### Background of the Invention

Biosurfactants are surfactants that are produced by mainly microorganisms and have distinctive functionality, and hence are gaining attention in many fields in recent years. The biosurfactants are roughly classified into a glycolipid type, a fatty acid type, a peptide type, a polymer type, and so on. In particular, the glycolipid type biosurfactants are highly useful in terms of productivity and functionality, and therefore various developments are also being carried out centering on the glycolipid type biosurfactants.

For example, Patent Literature 1 discloses a composition containing a biosurfactant, such as a glycolipid type and surfactin, and a carboxybetaine polymer, and attempts are being made to produce stable deposits on keratin materials such as the skin, a scalp, and hair. Patent Literature 2 discloses the use of a rhamnolipid for attaching a material such as a cationic polymer through a medium to the surface of the hair or the like, and such attachment improves the ease of detangling and combing of hair when the material is applied to hair as a shampoo compound.

Patent Literature 1: JP-A-2021-6509
Patent Literature 2: JP-A-2022-523244

### Summary of the Invention

The present invention provides a hair cleansing composition containing the following components (A) and (B):
(A) a glycolipid type biosurfactant; and
(B) an internal olefin sulfonic acid having 16 carbon atoms or a salt thereof, prepared by sulfonation of a raw material olefin having 16 carbon atoms and having an average double bond position of 3.9-position or more and 4.4-position or less.

Even in both Patent Literatures, improvements in foaming in application to hair and feeling of hair at the time of rinsing have not been yet sufficiently studied. In addition, in these Patent Literatures, there is no focus on the increasing risk of deteriorating the low-temperature stability of a composition with an increase in the order of structure of the glycolipid type biosurfactant and an increase in the length of the hydrophobic chain, and there is still room for improvement.

Accordingly, the present invention relates to a hair cleansing composition which uses a glycolipid type biosurfactant, exerts high performance when applied to hair, and also has excellent low-temperature stability.

The present inventor conducted various studies and found that a hair cleansing composition exerting excellent hair washing effect and expressing good low-temperature stability can be prepared by using a glycolipid type biosurfactant and a specific internal olefin sulfonic acid or a salt, prepared by sulfonation of a raw material olefin having an average double bond position within a very limited range.

The hair cleansing composition of the present invention when applied to hair provides good foaming and smooth foam quality at the time of washing, and softness and less friction feeling of the hair can be realized at the time of rinsing. The composition also has excellent low-temperature stability and is a composition having high utility.

The "good foaming" at the time of washing hair by the hair cleansing composition of the present invention means a sufficient volume of foam for realizing foaming power of forming quickly when washing hair and effect of removing dirt. The "smooth foam quality" at the time of washing hair by the hair cleansing composition of the present invention means refreshing and soft foaming and also smooth foam quality of allowing to realize the protective effect spread from the root to the tip of the hair.

Furthermore, the "softness of hair" at the time of rinsing after washing of hair by the hair cleansing composition of the present invention means not only being capable of realizing softness and suppleness of the whole hair at the time of rinsing but also being capable of realizing flexibility and healthiness of each strand of hair at the time of finishing.

"Less friction feeling of hair" at the time of rinsing means not only that excessive adhesion and remaining of a hair cleansing composition on the hair surface are suppressed and smooth texture of the hair surface without any foreign body sensation and discomfort can be felt but also that each strand of hair is prevented from unnecessarily tangling at the time of rinsing and smooth finger passage and feeling from roots to tips of hair and between tresses can be actually felt.

Here, "less friction feeling of hair" at the time of rinsing can be evaluated using, as an index, the amount of the hair cleansing composition adhering to the hair surface after application of the hair cleansing composition, followed by washing and rinsing. The adhesion amount is specifically the "coacervation amount (g/100 mL)" (the adhesion amount (g) on the hair surface per 100 mL of the hair cleansing composition), the amount of the hair cleansing composition adhering to the hair surface becomes excessive with an increase in the measured coacervation amount, and unpleasant feeling of remaining is increased and the friction feeling of hair is also increased.

In the hair cleansing composition of the present invention, since the coacervation amount can be controlled within an appropriate amount so that the protection effect on hair can be perceived, it is possible to effectively decrease the friction feeling at the time of rinsing which occurs by an excessive increase of the coacervation amount.

Hereinafter, the effects of these "good foaming", "smooth foam quality", "addition of softness to hair at the time of rinsing", and "addition of less friction feeling of hair at the time of rinsing" when the hair cleansing composition of the present invention is applied to hair are also collectively called "hair washing effects".

The "hair cleansing composition" of the present invention means a composition for washing not only hair but also a scalp and encompasses a composition for washing a head decoration product, "head decoration product cleansing composition".

Here, the "hair" means mainly human hair and also encompasses hair removed from a human head. The "head decoration product" means, for example, a hair wig, a wig, weaving, a hair extension, braided hair, a hair accessory, and doll hair, and the "head decoration product fiber" means a fiber that is used in such a head decoration product.

Accordingly, the objects to which the hair cleansing composition of the present invention is applied are hair, a scalp, and a head decoration product including a head decoration product fiber.

### Detailed Description of the Invention

The present invention will now be described in detail.

The hair cleansing composition of the present invention contains a glycolipid type biosurfactant as a component (A). The biosurfactant is roughly classified based on the structure of the hydrophilic group into a glycolipid type, a fatty acid type, a peptide type, and a polymer type. In the present invention, a glycolipid type biosurfactant composed of a sugar chain and a lipid is used.

That is, the hair cleansing composition of the present invention contains a specific internal olefin sulfonic acid or a salt thereof as a component (B) described later together with the glycolipid type biosurfactant as the component (A) and thereby exerts a synergistic effect of the characteristic structures and performances of both components, appropriately controls the coacervation amount while enabling expression of good foaming and smooth foam quality at the time of washing, effectively decreases the friction feeling at the time of rinsing, and can exert excellent hair washing effects. In addition, it is possible to secure good low-temperature storage stability by effectively suppressing unnecessary precipitation or the like. Specifically, in the hair cleansing composition of the present invention, the solubility of the component (B) is improved by blending the glycolipid type biosurfactant as the component (A), excessive precipitation of a coacervate can be suppressed, and good low-temperature storage stability can be secured.

Specifically, the component (A) can be one or more selected from the group consisting of a rhamnolipid, a sophorolipid, a trehalose lipid, and a mannosylalditol lipid. These components (A) may be protonated or may form a salt.

The rhamnolipid has a structure in which long-chain hydroxy fatty acid is bonded to rhamnose and is of the following formula (1): 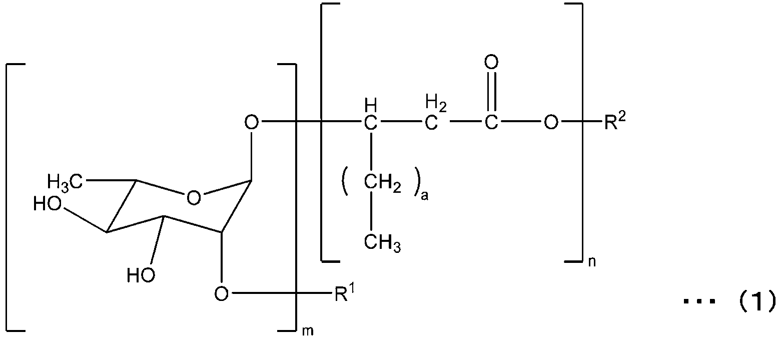 (in the formula (1), m and n are respectively independently an integer of 1 or 2; a is an integer of 4 or more and 10 or less; R¹ is H or CH₃(CH₂)_{b}CH=CHCO-; b is an integer of 4 or more and 10 or less; and R² is H or a cation).

As the rhamnolipid, for example, a marketed product, such as REHANCE (registered trademark) One (manufactured by Evonik Industries AG), can be used.

The sophorolipid has a structure in which long-chain hydroxy fatty acid is bonded to sophorose and can exist in a lactone type (LSL) formed of a cyclic ester bond between the carboxyl group of the long-chain hydroxy fatty acid and the hydroxy group of the sophorose, and in an acid type (ASL) prepared by hydrolyzation thereof.

The lactone type sophorolipid is of the following formula (2), and the acid type sophorolipid is of the following formula (3): 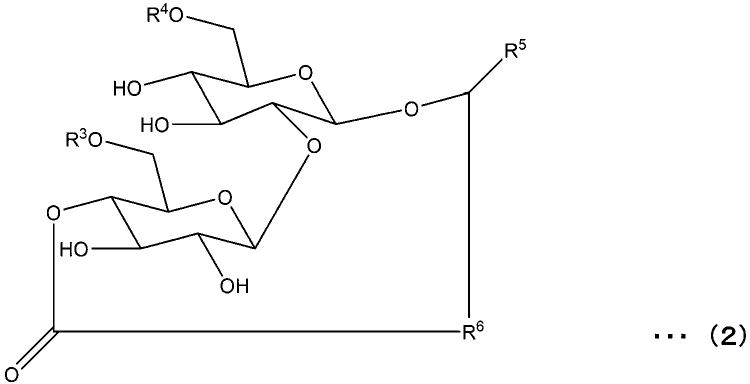 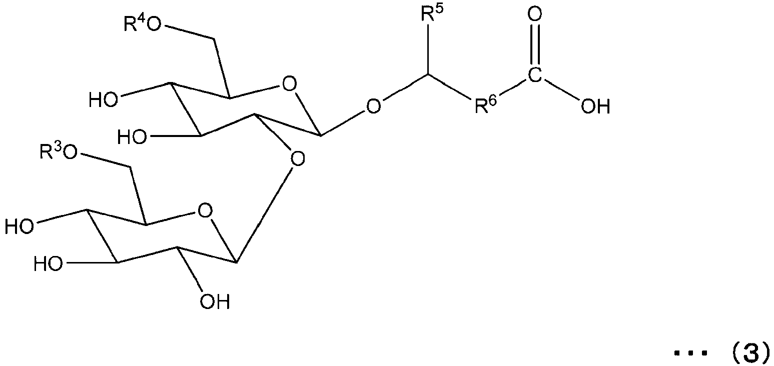 (in the formulae (2) and (3), R³, R⁴, R⁵, and R⁶ are synonymous; R³ and R⁴ are each independently H or an acetyl group; R⁵ is a saturated or unsaturated hydrocarbon group having 1 or more and 9 or less carbon atoms; and R⁶ is a saturated or unsaturated hydrocarbon group having 1 or more and 19 or less carbon atoms).

As the sophorolipid, for example, a marketed product, such as BioToLife (registered trademark) (manufactured by BASF SE), can be used.

The trehalose lipid is composed of trehalose and a fatty acid or an acid and is of the following formula (4): 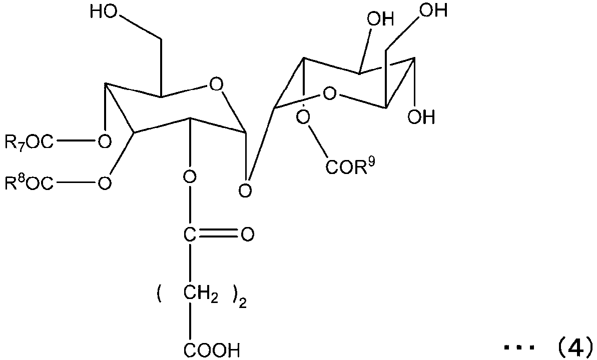 (in the formula (4), R⁷, R⁸, and R⁹ are each independently a saturated or unsaturated hydrocarbon group having 5 or more and 13 or less carbon atoms).

The mannosylalditol lipid has a structure in which a sugar alcohol is bonded to mannose, and examples thereof include a mannosylerythritol lipid (MEL), a mannosylmannitol lipid (MML), a mannosylsorbitol lipid (MSL), a mannosylarabitol lipid (MAraL), and a mannosylribitol lipid (MRL). Among them, preferred is a mannosylerythritol lipid of the following formula (5): 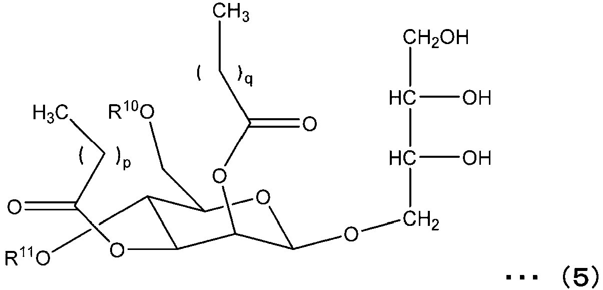 (in the formula (5), R¹⁰ and R¹¹ are each independently H or an acetyl group; and p and q are each independently an integer of 1 or 2).

The component (A) is preferably one or more selected from the group consisting of a rhamnolipid and a sophorolipid from the viewpoint of effectively decreasing the friction feeling at the time of rinsing and exerting excellent hair washing effects, and a rhamnolipid and a sophorolipid can be used in combination.

The content of the component (A) in the hair cleansing composition of the present invention is, from the viewpoint of effectively decreasing the friction feeling at the time of rinsing and effectively promoting the exertion of the excellent hair washing effects, preferably 0.05 mass% or more, more preferably 0.1 mass% or more, and further preferably 0.5 mass% or more. In addition, the content of the component (A) in the hair cleansing composition of the present invention is, from the viewpoint of securing good low-temperature stability while securing the expression of good foaming and smooth foam quality at the time of washing, preferably 4 mass% or less, more preferably 3 mass% or less, and further preferably 2 mass% or less. The content of the component (A) in the hair cleansing composition of the present invention is preferably 0.05 mass% or more and 4 mass% or less, more preferably from 0.05 to 3 mass%, further preferably from 0.1 to 3 mass%, further preferably from 0.1 to 2 mass%, and further more preferably from 0.5 to 2 mass%.

When a rhamnolipid is used as the component (A), the content of the rhamnolipid in the component (A) is preferably 50 mass% or more, more preferably 70 mass% or more, and further preferably 90 mass% or more and may be 100 mass%.

The hair cleansing composition of the present invention contains, as the component (B), an internal olefin sulfonic acid having 16 carbon atoms or a salt, prepared by sulfonation of a raw material olefin having 16 carbon atoms and having an average double bond position of 3.9-position or more and 4.4-position or less. That is, the internal olefin sulfonic acid having 16 carbon atoms or a salt thereof as the component (B) is a compound that is prepared by sulfonating a raw material olefin having an average double bond position within a specific, very limited range as a starting material, specifically, by sulfonating a raw material olefin and then performing neutralization and hydrolysis.

Examples of the salt of the internal olefin sulfonic acid having 16 carbon atoms prepared by sulfonation of a raw material olefin having 16 carbon atoms and having an average double bond position of 3.9-position or more and 4.4-position or less include one or more selected from the group consisting of alkali metal salts, such as a sodium salt and a potassium salt; organic amine salts, such as an ammonium salt, a monoethanolamine salt, a diethanolamine salt, a triethanolamine salt, a 2-aminoethanol salt, and a 2-aminomethyl propanediol salt; and basic amino acid salts, such as a lysine salt and an arginine salt. These internal olefin sulfonates having 16 carbon atoms may not be necessarily in a salt form from the beginning and may be a salt that is generated by a neutralization reaction during manufacturing.

In particular, the salt of the internal olefin sulfonic acid having 16 carbon atoms is, from the viewpoint of improving the low-temperature stability, preferably one or more selected from the group consisting of a sodium salt, a potassium salt, an ammonium salt, and a 2-aminoethanol salt, more preferably one or two selected from the group consisting of a sodium salt and a potassium salt, and further more preferably a sodium salt. That is, sodium internal olefin sulfonate having 16 carbon atoms is further more preferred.

The internal olefin sulfonic acid having 16 carbon atoms or a salt thereof as the component (B), which is a product obtained from such a raw material olefin having 16 carbon atoms, is mainly a mixture of hydroxyalkanesulfonic acid having 16 carbon atoms or a salt thereof (hydroxy form, abbreviation: "HAS") and olefin sulfonic acid having 16 carbon atoms or a salt thereof (olefin form, abbreviation: "IOS").

In the raw material olefin having 16 carbon atoms as a starting raw material as the component (B), the positions of double bonds are mainly located inside the carbon chain, but a so-called α-olefin in which the double bond is located at the 1-position of the carbon chain may be contained in a trace amount. When such a raw material olefin is sulfonated, β-sultone is mainly produced, and a part of the β-sultone is converted into γ-sultone and an olefin sulfonic acid. These β-sultone, γ-sultone, and olefin sulfonic acid are further converted into hydroxyalkanesulfonic acid having 16 carbon atoms or a salt thereof and olefin sulfonic acid having 16 carbon atoms or a salt thereof in the neutralization/hydrolysis process (for example, J. Am. Oil Chem. Soc. 69, 39 (1992)). The hydroxy group of the resulting hydroxyalkanesulfonic acid or a salt thereof is located inside the alkane chain, and the double bond of the olefin sulfonic acid or a salt thereof is located inside the olefin chain.

Accordingly, in the present specification, these products and a mixture thereof are collectively referred to as an internal olefin sulfonic acid having 16 carbon atoms or a salt thereof as the component (B).

The raw material olefin having 16 carbon atoms that is sulfonated and forms an internal olefin sulfonic acid having 16 carbon atoms or a salt thereof as the component (B) has an average double bond position of 3.9-position or more and 4.4-position or less. The raw material olefin having 16 carbon atoms and having an average double bond position of such a value has a broad distribution of double bond positions over from the 2-position to the 8-position including the 1-position that can be contained in a trace amount.

The double bond positions and its distribution in a raw material olefin can be verified by measurement using a gas chromatography mass spectrometer (abbreviation: "GC-MS"). Specifically, each of components having different carbon chain lengths and double bond positions is precisely separated by a gas chromatography apparatus (hereinafter, abbreviated as GC), and the double bond positions can be identified by application of them to a mass spectrometer (abbreviation: "MS"), and the distribution is determined from the respective GC peak areas.

On the other hand, in the internal olefin sulfonic acid having 16 carbon atoms or a salt thereof as the component (B) prepared by sulfonation of such a raw material olefin, the more the position of the sulfonate group introduced by sulfonation is inside the carbon chain, the more difficult the separation. Accordingly, there is no definitive analytical method at present. However, the positions of the sulfonate groups in the component (B) are fully presumed to approximately correspond to the double bond positions in the raw material olefin and to show a broad distribution over from the 2-position to the 8-position including the 1-position without being excessively unevenly distributed. Accordingly, in the present invention, the component (B) is regulated based on the value of the average double bond position in the raw material olefin as a starting raw material.

The component (B) used in the present invention is an internal olefin sulfonic acid or a salt, prepared from a raw material olefin having the above-mentioned average double bond position value, i.e., a broad double bond distribution, and the sulfonate groups in the carbon chain are present in broad positions without being excessively unevenly distributed. In an internal olefin sulfonic acid having 16 carbon atoms or a salt thereof, if sulfonate groups are unevenly distributed near the end of the carbon chain, the melting point rises by an increase of the lengthened carbon chains, and precipitation easily occurs. Accordingly, the low-temperature stability may be deteriorated. If sulfonate groups are unevenly distributed near the end of the carbon chain, coacervates tend to be excessively formed, the feeling of hair at the time of rinsing is deteriorated, and the hair washing effects may be decreased. However, in the internal olefin sulfonic acid having 16 carbon atoms or a salt thereof as the component (B) obtained from the above-mentioned raw material olefin, sulfonate groups are present in broad positions without being excessively unevenly distributed in the carbon chain, the internal olefin sulfonic acids or salts thereof having various lengths of the carbon chain from the bonding position of the sulfonate group to the end are moderately mixed. Accordingly, the coacervation amount is appropriately controlled together with the component (A) to effectively decrease the friction feeling at the time of rinsing, and good low-temperature storage stability can be expressed while maintaining the excellent hair washing effects. The same applies also when the component (B) is sodium internal olefin sulfonate having 16 carbon atoms.

The average double bond position (unit: position) in the raw material olefin having 16 carbon atoms as the starting raw material of the component (B) means the average value of the double bond positions of each raw material olefin having 16 carbon atoms present in the total amount of the raw material olefin having 16 carbon atoms. Specifically, the average double bond position of a raw material olefin having 16 carbon atoms is a value determined by the following expression (6).$\begin{matrix}Average double bond position of raw material \\ alefin having 16 carbon atoms\end{matrix} = {\sum }_{n = 1}^{8} n \times {C}_{n} / 100$

(In the expression (6), n represents an integer (unit: position) indicating the position of a double bond existing in a raw material olefin having 16 carbon atoms; and Cn represents the content (unit: mass%) of the raw material olefin having 16 carbon atoms and having a double bond at n-position in the total amount of 100 mass% of the raw material olefin having 16 carbon atoms.)

The average double bond position in the raw material olefin having 16 carbon atoms that forms the component (B) is 3.9-position or more and preferably 4.0-position or more, from the viewpoint of sufficiently enhancing the synergistic effect together with the component (A) and securing the expression of good low-temperature storage stability. The average double bond position in the raw material olefin having 16 carbon atoms is 4.4-position or less, preferably 4.3-position or less, and further preferably 4.2-position or less, from the viewpoint of appropriately controlling the coacervation amount together with the component (A) and securing the exertion of the excellent hair washing effects. In addition, the average double bond position in the raw material olefin having 16 carbon atoms is 3.9-position or more and 4.4-position or less, preferably from 4.0-position to 4.3-position, and more preferably from 4.0-position to 4.2-position.

In the raw material olefin having 16 carbon atoms, the content of a raw material olefin having a double bond position at the 2-position in the raw material olefin having 16 carbon atoms is preferably 10 mass% or more, more preferably 15 mass% or more, and further more preferably 20 mass% or more and preferably 35 mass% or less, more preferably 32 mass% or less, and further more preferably 24 mass% or less. In addition, in the raw material olefin having 16 carbon atoms, the content of the raw material olefin having a double bond position at the 2-position in the raw material olefin having 16 carbon atoms is preferably from 10 to 35 mass%, more preferably from 15 to 32 mass%, and further more preferably from 20 to 24 mass%.

In the raw material olefin having 16 carbon atoms, the content of a raw material olefin having a double bond position at the 3-position in the raw material olefin having 16 carbon atoms is preferably 10 mass% or more, more preferably 14 mass% or more, and further more preferably 16 mass% or more and preferably 30 mass% or less, more preferably 24 mass% or less, and further more preferably 19 mass% or less. In addition, in the raw material olefin having 16 carbon atoms, the content of the raw material olefin having a double bond position at the 3-position in the raw material olefin having 16 carbon atoms is preferably from 10 to 30 mass%, more preferably from 14 to 24 mass%, and further more preferably from 16 to 19 mass%.

In the raw material olefin having 16 carbon atoms, the content of a raw material olefin having a double bond position at the 4-position in the raw material olefin having 16 carbon atoms is preferably 10 mass% or more, more preferably 15 mass% or more, and further more preferably 17 mass% or more and preferably 30 mass% or less, more preferably 25 mass% or less, and further more preferably 19 mass% or less. In addition, in the raw material olefin having 16 carbon atoms, the content of the raw material olefin having a double bond position at the 4-position in the raw material olefin having 16 carbon atoms is preferably from 10 to 30 mass%, more preferably from 15 to 25 mass%, and further more preferably from 17 to 19 mass%.

In the raw material olefin having 16 carbon atoms, the content of a raw material olefin having a double bond position at the 5-position in the raw material olefin having 16 carbon atoms is preferably 5 mass% or more, more preferably 10 mass% or more, and further more preferably 13 mass% or more and preferably 25 mass% or less, more preferably 19 mass% or less, and further more preferably 15 mass% or less. In addition, in the raw material olefin having 16 carbon atoms, the content of the raw material olefin having a double bond position at the 5-position is preferably from 5 to 25 mass%, more preferably from 10 to 19 mass%, and further more preferably from 13 to 15 mass% in the raw material olefin having 16 carbon atoms.

In the raw material olefin having 16 carbon atoms, the content of a raw material olefin having a double bond position at the 6-position in the raw material olefin having 16 carbon atoms is preferably 5 mass% or more, more preferably 7 mass% or more, and further more preferably 11 mass% or more and preferably 20 mass% or less, more preferably 15 mass% or less, and further more preferably 13 mass% or less. In addition, in the raw material olefin having 16 carbon atoms, the content of the raw material olefin having a double bond position at the 6-position in the raw material olefin having 16 carbon atoms is preferably from 5 to 20 mass%, more preferably from 7 to 15 mass%, and further more preferably from 11 to 13 mass%.

In the raw material olefin having 16 carbon atoms, the total content of raw material olefins having a double bond position at the 7-position or 8-position in the raw material olefin having 16 carbon atoms is preferably 5 mass% or more, more preferably 7 mass% or more, and further more preferably 12 mass% or more and preferably 25 mass% or less, more preferably 22 mass% or less, and further more preferably 16 mass% or less. In addition, in the raw material olefin having 16 carbon atoms, the total content of the raw material olefins having a double bond position at the 7-position or 8-position in the raw material olefin having 16 carbon atoms is preferably from 5 to 25 mass%, more preferably from 7 to 22 mass%, and further more preferably from 12 to 16 mass%.

In the raw material olefin having 16 carbon atoms, the mass ratio of the content of raw material olefins having a double bond position at the 3- to 5-positions to the content of raw material olefins having a double bond position at the 6- to 8-positions, (raw material olefin_{3 to 5-position}/raw material olefin_{6 to 8-position}), is preferably 1.0 or more, more preferably 1.3 or more, and further more preferably 1.7 or more and preferably 4.0 or less, more preferably 3.5 or less, and further more preferably 2.2 or less. In addition, in the raw material olefin having 16 carbon atoms, the mass ratio of the content of raw material olefins having a double bond position at the 3- to 5-positions to the content of raw material olefins having a double bond position at the 6- to 8-positions, (raw material olefin_{3 to 5-position}/raw material olefin_{6 to 8-position}), is preferably from 1.0 to 4.0, more preferably from 1.3 to 3.5, and further more preferably from 1.7 to 2.2.

In the raw material olefin having 16 carbon atoms, the content of a raw material olefin having a double bond position at the 1-position (α-olefin), which may inevitably exist, is preferably less than 5.0 mass%, more preferably less than 3.0 mass%, and further more preferably less than 2.5 mass% in the raw material olefin having 16 carbon atoms. Alternatively, the raw material olefin having 16 carbon atoms preferably does not contain α-olefin.

The above-mentioned raw material olefin having 16 carbon atoms can be prepared by isomerization (double bond transfer) of a raw material olefin having a double bond position at the 1-position (α-olefin) that is generated by dehydration reaction of an alcohol having 16 carbon atoms. Specifically, a solid acid catalyst, such as alumina, in an amount of preferably 0.5 parts by mass or more, more preferably 2 parts by mass or more, and preferably 15 parts by mass or less, more preferably 10 parts by mass or less, and preferably from 0.5 to 15 parts by mass, more preferably from 2 to 10 parts by mass, is added to 100 parts by mass of 1-hexadecanol.

Subsequently, isomerization reaction is performed by stirring at preferably 220°C or more, more preferably 260°C or more, and preferably 350°C or less, and preferably from 220°C to 350°C, more preferably from 260°C to 350°C, for preferably 1 hour or more, more preferably 3 hours or more, and preferably 30 hours or less, more preferably 10 hours or less, and preferably from 1 to 30 hours, more preferably from 3 to 10 hours. The product after completion of the reaction is appropriately distilled to obtain above-mentioned raw material olefin having 16 carbon atoms.

In an internal olefin sulfonic acid having 16 carbon atoms or a salt thereof as the component (B) prepared by sulfonation of the above-described raw material olefin having 16 carbon atoms, the content of the internal olefin sulfonic acid having a sulfonate group at the 1-position or more and the 4-position or less or a salt thereof in the component (B) is preferably 40 mass% or more, more preferably 50 mass% or more, and further more preferably 55 mass% or more and preferably 75 mass% or less, more preferably 70 mass% or less, and further more preferably 68 mass% or less. In addition, in the internal olefin sulfonic acid having 16 carbon atoms or a salt thereof as the component (B) prepared by sulfonation of the above-described raw material olefin having 16 carbon atoms, the content of the internal olefin sulfonic acid having a sulfonate group at the 1-position or more and the 4-position or less or a salt thereof in the component (B) is preferably 40 mass% or more and 75 mass% or less, more preferably from 50 to 70 mass%, and further more preferably from 55 to 68 mass%.

When the component (B) is sodium internal olefin sulfonate having 16 carbon atoms, the content of sodium internal olefin sulfonate having a sulfonate group at the 1-position or more and 4-position or less in the sodium internal olefin sulfonate having 16 carbon atoms is also same as above.

In the internal olefin sulfonic acid having 16 carbon atoms or a salt thereof as the component (B), the content of an internal olefin sulfonic acid having a sulfonate group at the 2-position or a salt thereof in the component (B) is preferably 10 mass% or more, more preferably 13 mass% or more, and further more preferably 17 mass% or more and preferably 35 mass% or less, more preferably 30 mass% or less, and further more preferably 25 mass% or less. In addition, in the internal olefin sulfonic acid having 16 carbon atoms or a salt thereof as the component (B), the content of the internal olefin sulfonic acid having a sulfonate group at the 2-position or a salt thereof in the component (B) is preferably 10 mass% or more and 35 mass% or less, more preferably from 13 to 30 mass%, and further more preferably from 17 to 25 mass%.

When the component (B) is sodium internal olefin sulfonate having 16 carbon atoms, in the sodium internal olefin sulfonate having 16 carbon atoms, the content of sodium internal olefin sulfonate having a sulfonate group at the 2-position is also same as above.

In the internal olefin sulfonic acid having 16 carbon atoms or a salt thereof as the component (B), the content of an internal olefin sulfonic acid having a sulfonate group at the 3-position or a salt thereof in the component (B) is preferably 5 mass% or more, more preferably 11 mass% or more, and further more preferably 15 mass% or more and preferably 30 mass% or less, more preferably 25 mass% or less, and further more preferably 20 mass% or less. In addition, in the internal olefin sulfonic acid having 16 carbon atoms or a salt thereof as the component (B), the content of the internal olefin sulfonic acid having a sulfonate group at the 3-position or a salt thereof in the component (B) is preferably 5 mass% or more and 30 mass%, more preferably from 11 to 25 mass%, and further more preferably from 15 to 20 mass%.

When the component (B) is sodium internal olefin sulfonate having 16 carbon atoms, the content of sodium internal olefin sulfonate having a sulfonate group at the 3-position in the sodium internal olefin sulfonate having 16 carbon atoms is also same as above.

In the internal olefin sulfonic acid having 16 carbon atoms or a salt thereof as the component (B), the content of an internal olefin sulfonic acid having a sulfonate group at the 4-position or a salt thereof in the component (B) is preferably 15 mass% or more, more preferably 18 mass% or more, and further more preferably 19 mass% or more and preferably 30 mass% or less, more preferably 25 mass% or less, and further more preferably 23 mass% or less. In addition, in the internal olefin sulfonic acid having 16 carbon atoms or a salt thereof as the component (B), the content of the internal olefin sulfonic acid having a sulfonate group at the 4-position or a salt thereof in the component (B) is preferably 15 mass% or more and 30 mass% or less, more preferably from 18 to 25 mass%, and further more preferably from 19 to 23 mass%.

When the component (B) is sodium internal olefin sulfonate having 16 carbon atoms, in the sodium internal olefin sulfonate having 16 carbon atoms, the content of sodium internal olefin sulfate having a sulfonate group at the 4-position is also same as above.

In the internal olefin sulfonic acid having 16 carbon atoms or a salt thereof as the component (B), the content of an internal olefin sulfonic acid having a sulfonate group at the 1-position or a salt thereof in the component (B) is preferably less than 5.0 mass%, more preferably less than 3.0 mass%, and further more preferably less than 2.5 mass%. Alternatively, the internal olefin sulfonic acid having 16 carbon atoms or a salt thereof as the component (B) preferably does not contain the internal olefin sulfonic acid having a sulfonate group at the 1-position or a salt thereof.

When the component (B) is sodium internal olefin sulfonate having 16 carbon atoms, the content of sodium internal olefin sulfonate having a sulfonate group at the 1-position in the sodium internal olefin sulfonate having 16 carbon atoms is also same as above. Alternatively, it is preferable that the sodium internal olefin sulfonate having a sulfonate group at the 1-position is not contained.

In the internal olefin sulfonic acid having 16 carbon atoms or a salt thereof as the component (B), the mass ratio of the content of the hydroxy form (HAS) to the content of the olefin form (IOS), hydroxy form/olefin form, is preferably from 50/50 to 100/0, more preferably from 60/40 to 100/0, further more preferably from 70/30 to 100/0, further more preferably from 75/25 to 100/0, and more preferably from 75/25 to 95/5 from the viewpoint of productivity improvement and impurity reduction.

Such a mass ratio (hydroxy form/olefin form) is determined based on HPLC-MS peak areas that are prepared by separation of the hydroxy form and the olefin form from the component (A) by HPLC and application to MS.

When the component (B) is sodium internal olefin sulfonate having 16 carbon atoms, the mass ratio of the content of the hydroxy form (HAS) to the content of the olefin form (IOS), hydroxy form/olefin form, in the sodium internal olefin sulfonate having 16 carbon atoms is also same as above.

Since the internal olefin sulfonic acid having 16 carbon atoms or a salt thereof as the component (B) can be prepared by sulfonation of a raw material olefin, there is a possibility that the unreacted raw material olefin and the inorganic compound remain in the component (B). Lower contents of these components are preferable. The same applies also when the component (B) is sodium internal olefin sulfonate having 16 carbon atoms.

In the internal olefin sulfonic acid having 16 carbon atoms or a salt thereof as the component (B), the content of the unreacted raw material olefin in the component (B) is preferably less than 5.0 mass%, more preferably less than 3.0 mass%, further more preferably less than 1.5 mass%, and further more preferably less than 1.0 mass%.

When the component (B) is sodium internal olefin sulfonate having 16 carbon atoms, the content of the unreacted raw material olefin in the sodium internal olefin sulfonate having 16 carbon atoms is also same as above.

In the internal olefin sulfonic acid having 16 carbon atoms or a salt thereof as the component (B), the content of the inorganic compound in the component (B) is preferably less than 7.5 mass%, more preferably less than 5.0 mass%, further more preferably less than 3.0 mass%, further more preferably less than 2.0 mass%, and even more preferably less than 1.6 mass%.

When the component (B) is sodium internal olefin sulfonate having 16 carbon atoms, the content of the inorganic compound in the sodium internal olefin sulfonate having 16 carbon atoms is also same as above.

The content of the component (B) in the hair cleansing composition of the present invention is, from the viewpoint of effectively promoting the exertion of the excellent hair washing effects while effectively decreasing the friction feeling at the time of rinsing together with the component (A), preferably 0.01 mass% or more, more preferably 0.05 mass% or more, further preferably 0.1 mass% or more, further preferably 0.5 mass% or more, and further more preferably 2 mass% or more. The content of the component (B) in the hair cleansing composition of the present invention is preferably 30 mass% or less, more preferably 15 mass% or less, further more preferably 12 mass% or less, further more preferably 10 mass% or less, and even more preferably 8 mass% or less from the viewpoint of securing good low-temperature stability. In addition, the content of the component (B) in the hair cleansing composition of the present invention is preferably 0.01 mass% or more and 30 mass% or less, more preferably from 0.05 to 15 mass%, further more preferably from 0.1 to 12 mass%, further more preferably from 0.5 to 12 mass%, even more preferably from 2 to 12 mass%, even more preferably from 2 to 10 mass%, and even more preferably from 2 to 8 mass%.

The internal olefin sulfonic acid having 16 carbon atoms or a salt thereof as the component (B) can be obtained through sulfonation by reacting the raw material olefin having 16 carbon atoms with sulfur trioxide, specifically, by sulfonating the raw material olefin and then performing neutralization and subsequently hydrolysis.

More specifically, the amount of the sulfur trioxide that is used for sulfonation of the raw material olefin is preferably 0.8 mol or more, more preferably 0.9 mol or more, and further more preferably 0.95 mol or more based on 1 mol of the raw material olefin from the viewpoint of improving the yield of the component (B) and the viewpoint of improving the reactivity. In addition, the amount of the sulfur trioxide that is used for sulfonation of the raw material olefin is preferably 1.2 mol or less, more preferably 1.1 mol or less, and further more preferably 1.05 mol or less from the viewpoint of the economic efficiency and the viewpoint of suppressing unnecessary coloring of the component (B). In addition, the amount of the sulfur trioxide that is used for sulfonation of the raw material olefin is preferably from 0.8 to 1.2 mol, more preferably from 0.9 to 1.1 mol, and further more preferably from 0.95 to 1.05 mol based on 1 mol of the raw material olefin.

The reaction temperature when the raw material olefin is sulfonated is preferably 0°C or more from the viewpoint of preventing coagulation of the sulfur trioxide and the component (B) and is preferably 50°C or less from the viewpoint of suppressing unnecessary coloring of the component (B). The reaction temperature when the raw material olefin is sulfonated is preferably from 0°C to 50°C.

In the neutralization, an alkali compound, such as sodium hydroxide, potassium hydroxide, ammonia, and 2-aminoethanol, is used for the reaction. The amount of the alkali compound to be added is preferably 1.0 times mol or more, more preferably 1.03 times mol or more, per 1 mol of the sulfonate group from the viewpoint of suppressing the generation of impurities such as a raw material olefin and an inorganic salt and from the viewpoint of improving the reactivity. In addition, the amount of the alkali compound to be added is preferably 2.5 times mol or less, more preferably 2.0 times mol or less, and further more preferably 1.5 times mol or less per 1 mol of the sulfonate group from the viewpoint of the economic efficiency and the viewpoint of suppressing the generation of impurities such as a raw material olefin and an inorganic salt. The amount of the alkali compound to be added is preferably from 1.0 to 2.5 times mol, more preferably from 1.03 to 2.0 times mol, and further more preferably from 1.03 to 1.5 times mol per 1 mol of the sulfonate group.

In the neutralization, the temperature when the sulfonated raw material olefin and an alkali compound are mixed and the reaction temperature are preferably 40°C or less, more preferably 35°C or less, further more preferably 30°C or less, and even more preferably 25°C or less from the viewpoint of suppressing the generation of impurities, such as an internal olefin and an inorganic salt, by side reaction and is preferably 0°C or more, more preferably 10°C or more, further more preferably 15°C or more, and even more preferably 20°C or more from the viewpoint of improving the reactivity. In addition, the temperature when the sulfonated raw material olefin and an alkali compound are mixed and the reaction temperature are preferably from 0°C to 40°C, more preferably from 10°C to 35°C, further more preferably from 15°C to 30°C, and even more preferably from 20°C to 25°C.

The reaction temperature for hydrolysis that is performed after neutralization is preferably 120°C or more, more preferably 140°C or more, and further more preferably 160°C or more from the viewpoint of improving the reactivity in the presence of water. In addition, the reaction temperature for the hydrolysis is preferably 220°C or less and more preferably 180°C or less from the viewpoint of suppressing decomposition of the product. In addition, the reaction temperature for the hydrolysis is preferably from 120°C to 220°C, more preferably from 140°C to 180°C, and further more preferably from 160°C to 180°C.

The reaction time for the hydrolysis is preferably 30 minutes or more and more preferably 45 minutes or more from the viewpoint of completing the reaction. The reaction time for the hydrolysis is preferably 240 minutes or less, more preferably 180 minutes or less, further more preferably 120 minutes or less, and even more preferably 90 minutes or less from the viewpoint of improving the productivity. In addition, the reaction time for the hydrolysis is preferably from 30 to 240 minutes, more preferably from 45 to 180 minutes, further more preferably from 45 to 120 minutes, and even more preferably from 45 to 90 minutes. These reactions can be performed in series. After the completion of the reaction, purification can be performed by extraction, washing, and so on.

The same applies also when sodium internal olefin sulfonate having 16 carbon atoms is obtained as the component (B).

The mass ratio of the content of the component (A) to the content of the component (B), (A)/(B), is preferably 0.005 or more, more preferably 0.01 or more, and further preferably 0.05 or more, from the viewpoint of appropriately controlling the coacervation amount and effectively decreasing the friction feeling at the time of rinsing while securing the expression of good foaming and smooth foam quality at the time of washing and effectively exerting the excellent hair washing effects. In addition, the mass ratio of the content of the component (A) to the content of the component (B), (A)/(B), is preferably 0.60 or less, more preferably 0.50 or less, and further preferably 0.30 or less, from the viewpoint of effectively suppressing unnecessary precipitation due to the component (A) and the component (B) and exerting good low-temperature stability. The mass ratio of the content of the component (A) to the content of the component (B), (A)/(B), is preferably 0.005 or more and 0.60 or less, more preferably from 0.005 to 0.50, further preferably from 0.01 to 0.50, further more preferably from 0.01 to 0.30, and further more preferably from 0.05 to 0.30.

The mass ratio of the content of the component (A) to the total content of the component (A) and the component (B), (A)/{(A)+(B)}, is preferably 0.001 or more, more preferably 0.01 or more, further preferably 0.02 or more, and further more preferably 0.05 or more and preferably 0.5 or less, more preferably 0.35 or less, and further preferably 0.25 or less, from the viewpoint of appropriately controlling the coacervation amount and effectively decreasing the friction feeling at the time of rinsing while securing the expression of good foaming and smooth foam quality at the time of washing and effectively exerting the excellent hair washing effects. The mass ratio of the content of the component (A) to the total content of the component (A) and the component (B), (A)/{(A)+(B)}, is preferably 0.001 or more and 0.5 or less, more preferably from 0.01 to 0.35, further preferably from 0.02 to 0.25, and further more preferably from 0.05 to 0.25.

The hair cleansing composition of the present invention contains an anionic surfactant other than the component (B), as a component (C). The exertion of the excellent hair washing effects by the synergistic effect of the component (A) and the component (B) is promoted and good low-temperature stability can be secured by containing the component (C).

Specifically, examples of the component (C) include one or more selected from the group consisting of a sulfonate, an amino acid salt, a sulfosuccinate, a sulfuric ester salt, and a carboxylate.

The sulfonate as the component (C) is a sulfonate other than the component (B), and more specifically, examples of the sulfonate include one or more selected from the group consisting of aminoethyl sulfonates such as N-acylmethyltaurate, alkyl benzene sulfonates, alkenyl benzene sulfonates, alkane sulfonates, and α-olefin sulfonates having 14 carbon atoms.

More specifically, examples of the amino acid salt include one or more selected from the group consisting of acylglutamates, sarcosine derivatives, alanine derivatives, glycine derivatives, and arginine derivatives.

Examples of the sulfosuccinate include one or more selected from the group consisting of sulfosuccinic acid alkyl ester salts and polyoxyalkylene sulfosuccinic acid alkyl ester salts.

Examples of the sulfuric ester salt include one or more selected from the group consisting of alkyl sulfates, alkenyl sulfates, polyoxyalkylene alkyl ether sulfates, polyoxyalkylene alkenyl ether sulfates, polyoxyalkylene alkylphenyl ether sulfates and so on.

Examples of the carboxylate include one or more selected from the group consisting of fatty acid salts, polyoxyalkylene alkyl ether acetates, and so on.

In particular, from the viewpoint of achieving both the exertion of excellent hair washing effect and the expression of good low-temperature stability, one or more selected from the group consisting of aminoethyl sulfonate, amino acid salts, sulfosuccinates, polyoxyalkylene alkyl ether sulfates, and polyoxyalkylene alkyl ether acetates are preferable; one or more selected from the group consisting of aminoethyl sulfonate, amino acid salts, sulfosuccinates, and polyoxyalkylene alkyl ether sulfates are more preferable; and aminoethyl sulfonate is further preferable.

The content of the component (C) in the hair cleansing composition of the present invention is preferably 0.01 mass% or more, more preferably 0.05 mass% or more, further more preferably 0.1 mass% or more, further more preferably 0.8 mass% or more, and even more preferably 1.5 mass% or more from the viewpoint of effectively promoting the exertion of excellent hair washing effect by the synergistic effect of the component (A) and the component (B). The content of the component (C) in the hair cleansing composition of the present invention is preferably 30 mass% or less, more preferably 15 mass% or less, further more preferably 12 mass% or less, further more preferably 10 mass% or less, even more preferably 9.3 mass% or less, and even more preferably 8.0 mass% or less from the viewpoint of securing good low-temperature stability. In addition, the content of the component (C) in the hair cleansing composition of the present invention is preferably 0.01 mass% or more and 30 mass% or less, more preferably from 0.05 to 15 mass%, further more preferably from 0.1 to 12 mass%, further more preferably from 0.8 to 10 mass%, even more preferably from 0.8 to 9.3 mass%, even more preferably from 0.8 to 8.0 mass%, and even more preferably from 1.5 to 8.0 mass%.

The mass ratio of the content of the component (B) to the content of the component (C), (B)/(C), is preferably 0.01 or more, more preferably 0.05 or more, further preferably 0.1 or more, and further preferably 0.3 or more, from the viewpoint of effectively suppressing the unnecessary precipitation due to the component (A) and the component (B) and exerting good low-temperature stability. The mass ratio of the content of the component (B) to the content of the component (C), (B)/(C), is preferably 100 or less, more preferably 25 or less, further preferably 15 or less, further preferably 10 or less, and further more preferably 8 or less, from the viewpoint of effectively exerting also the excellent hair washing effects. In addition, the mass ratio of the content of the component (B) to the content of the component (C), (B)/(C), is preferably 0.01 or more and 100 or less, more preferably from 0.05 to 25, further preferably from 0.1 to 15, further preferably from 0.1 to 10, further more preferably from 0.3 to 10, and further more preferably from 0.3 to 8.

When the component (C) contains an internal olefin sulfonic acid or a salt thereof other than those having 16 carbon atoms, from the viewpoint of improving the smoothness of foam and low-temperature stability, the mass ratio of the content of the component (B) to the total amount of the internal olefin sulfonic acids or salts thereof in the hair cleansing composition of the present invention, (B)/(total amount of internal olefin sulfonic acids or salts thereof), is preferably 0.2 or more, more preferably 0.4 or more, further more preferably 0.6 or more, even more preferably 0.8 or more, even more preferably 0.85 or more, even more preferably 0.9 or more, and even more preferably 0.95 or more.

The hair cleansing composition of the present invention can contain an amphoteric surfactant (D) from the viewpoint of further ensuring the exertion of excellent hair washing effect and the expression of good low-temperature stability by the synergistic effect of the component (A) and the component (B).

Specifically, examples of the component (D) include one or more selected from the group consisting of carbobetaines and sulfobetaines. More specifically, the examples include one or more selected from the group consisting of carbobetaines and sulfobetaines each including an alkyl group, alkenyl group, or acyl group having from 6 to 22 carbon atoms and preferably from 8 to 18 carbon atoms.

In particular, one or more selected from the group consisting of lauramidopropyl betaine, cocamidopropyl betaine, lauramidopropyl hydroxysulfobetaine, and lauryl sulfobetaine are preferable, and one or two selected from the group consisting of lauramidopropyl betaine and lauramidopropyl hydroxysulfobetaine are more preferable.

The content of the component (D) in the hair cleansing composition of the present invention is preferably 0.01 mass% or more, more preferably 0.05 mass% or more, and further more preferably 0.1 mass% or more from the viewpoint of further enhancing the exertion of excellent hair washing effect. The content of the component (D) in the hair cleansing composition of the present invention is preferably 30 mass% or less, more preferably 15 mass% or less, and further more preferably 12 mass% or less from the viewpoint of further securing good low-temperature stability. In addition, the content of the component (D) in the hair cleansing composition of the present invention is preferably from 0.01 to 30 mass%, more preferably from 0.05 to 15 mass%, and further more preferably from 0.1 to 12 mass%.

The mass ratio of the content of the component (B) to the content of the component (D), (B)/(D), is preferably 0.01 or more, more preferably 0.05 or more, and further more preferably 0.1 or more from the viewpoint of effectively suppressing unnecessary precipitation due to the component (A) and the component (B) and exerting good low-temperature stability. The mass ratio of the content of the component (A) to the content of the component (C), (A)/(C), is preferably 50 or less, more preferably 25 or less, and further more preferably further more preferably 9 or less from the viewpoint of effectively enhancing excellent hair washing effect. In addition, the mass ratio of the content of the component (A) to the content of the component (C), (A)/(C), is preferably from 0.01 to 50, more preferably from 0.05 to 25, and further more preferably from 0.1 to 9.

The hair cleansing composition of the present invention can contain a nonionic surfactant (E) from the viewpoint of further ensuring the exertion of excellent hair washing effect and the expression of good low-temperature stability coupled with the above-mentioned components.

The component (E) is specifically one or more selected from the group consisting of a polyoxyalkylene alkyl ether, a fatty acid alkanolamide, a chemically synthesized alkyl glycoside, and an alkyl glyceryl ether.

More specifically, examples of the polyoxyalkylene alkyl ether include polyoxyalkylene alkyl ethers with an alkyl group having from 6 to 22 carbon atoms, preferably from 8 to 18 carbon atoms. In particular, preferred is a polyoxyethylene alkyl ether, more preferred is a polyoxyethylene alkyl ether with an oxyethylene group having an average number of moles added of from 3 to 50, and further preferred is a polyoxyethylene alkyl ether with an oxyethylene group having an average number of moles added of from 4 to 16.

Examples of the fatty acid alkanolamide include mono- or di-alkanolamide of which the fatty acid has from 6 to 22 carbon atoms, preferably from 8 to 18 carbon atoms.

Examples of the chemically synthesized alkyl glycoside include those of which the alkyl group has from 6 to 22 carbon atoms, preferably from 8 to 18 carbon atoms.

Examples of the alkyl glyceryl ether include those of which the alkyl group has from 6 to 22 carbon atoms, preferably from 8 to 18 carbon atoms.

In particular, fatty acid alkanolamide is more preferable.

The content of the component (E) in the hair cleansing composition of the present invention is preferably 0.01 mass% or more, more preferably 0.05 mass% or more, further more preferably 0.1 mass% or more, and further more preferably 0.5 mass% or more from the viewpoint of further enhancing the exertion of excellent hair washing effect by the synergistic effect of the component (A) and the component (B). The content of the component (E) in the hair cleansing composition of the present invention is preferably 15 mass% or less, more preferably 10 mass% or less, further more preferably 8 mass% or less, and further more preferably 6 mass% or less from the viewpoint of effectively securing good low-temperature stability. In addition, the content of the component (E) in the hair cleansing composition of the present invention is preferably from 0.01 to 15 mass%, more preferably from 0.05 to 10 mass%, further more preferably from 0.1 to 8 mass%, and further more preferably from 0.5 to 6 mass%.

The mass ratio of the content of the component (B) to the content of the component (E), (B)/(E), is preferably 0.01 or more, more preferably 0.05 or more, and further more preferably 0.1 or more from the viewpoint of effectively suppressing unnecessary precipitation due to the component (A) and the component (B) and exerting good low-temperature stability. The mass ratio of the content of the component (B) to the content of the component (E), (B)/(E), is preferably 50 or less, more preferably 25 or less, and further more preferably 9 or less from the viewpoint of effectively providing also excellent hair washing effect. In addition, the mass ratio of the content of the component (B) to the content of the component (E), (B)/(E), is preferably from 0.01 to 50, more preferably from 0.05 to 25, and further more preferably from 0.1 to 9.

The hair cleansing composition of the present invention can contain a cationic polymer (F) from the viewpoint of further ensuring the exertion of excellent hair washing effect and the expression of good low-temperature stability coupled with the above-mentioned components. Here, the term "cationic polymer" means a polymer having a substituent that is positively ionized when dissolved in water.

Specifically, examples of the component (F) include one or more selected from the group consisting of cationated polygalactomannane, cationated hydroxyalkyl cellulose, diallyl quaternary ammonium salt polymers, copolymers containing methacrylamidopropyltrimethylammonium chloride, and crosslinked cationic polymers.

More specifically, examples of the cationated polygalactomannane include one or more selected from the group consisting of cationated guar gum, cationated tara gum, and cationated locust bean gum.

More specifically, examples of the cationated hydroxyalkyl cellulose include one or two selected from the group consisting of cationated hydroxyethyl cellulose, cationated hydroxypropyl cellulose, and so on.

More specifically, examples of the diallyl quaternary ammonium salt polymer include one or more selected from the group consisting of polydiallyldimethylammonium chloride, diallyldimethylammonium chloride/acrylic acid copolymers, diallyldimethylammonium chloride/acrylamide copolymers, and diallyldimethylammonium chloride/acrylic acid/acrylamide copolymers.

Examples of the copolymer containing methacrylamidopropyltrimethylammonium chloride include one or more selected from the group consisting of acrylic acid/methyl acrylate/methacrylamidopropyltrimethylammonium chloride copolymers and acrylic acid/acrylamide/methacrylamidopropyltrimethylammonium chloride copolymers.

More specifically, examples of the crosslinked cationic polymer include N,N-dimethylaminoethyl methacrylate diethyl sulfate/N,N-dimethylacrylamide/dimethacrylate polyethylene glycol copolymers.

In particular, one or more selected from the group consisting of cationated hydroxyalkyl cellulose and crosslinked cationic polymers are preferable, and cationated hydroxyethyl cellulose is further preferable.

The content of the component (F) in the hair cleansing composition of the present invention is preferably 0.01 mass% or more, more preferably 0.05 mass% or more, and further more preferably 0.1 mass% or more from the viewpoint of effectively promoting the exertion of excellent hair washing effect. The content of the component (F) in the hair cleansing composition of the present invention is preferably 10 mass% or less, more preferably 3 mass% or less, and further more preferably 1 mass% or less from the viewpoint of securing good handling properties. In addition, the content of the component (F) in the hair cleansing composition of the present invention is preferably from 0.01 to 10 mass%, more preferably from 0.05 to 3 mass%, and further more preferably from 0.1 to 1 mass%.

The mass ratio of the content of the component (B) to the content of the component (F), (B)/(F), is preferably 1 or more, more preferably 5 or more, and further more preferably 10 or more from the viewpoint of exerting good handling properties of the component (B). The mass ratio of the content of the component (B) to the content of the component (F), (B)/(F), is preferably 1 000 or less, more preferably 500 or less, and further more preferably 100 or less from the viewpoint of effectively suppressing unnecessary precipitation due to the component (A) and the component (B) and exerting good low-temperature stability. In addition, the mass ratio of the content of the component (B) to the content of the component (F), (B)/(F), is preferably from 1 to 1 000, more preferably from 5 to 500, and further more preferably from 10 to 100.

The hair cleansing composition of the present invention can contain, in addition to the above-mentioned components, a viscosity reducer, polyhydric alcohols, a preservative, and a reducing agent, and also other components that are usually used as cosmetic raw materials, within range that do not impair the effects of the present invention. Examples of such components include a texture improver, a thickener, perfume, a UV absorber, a visible light absorber, a chelating agent, an antioxidant, a colorant, a preservative, a pH adjuster, a viscosity adjuster, a pearlescent agent, and a wetting agent.

The pH of the hair cleansing composition of the present invention at 25°C is, as the pH of a 5% water dispersion, preferably 3.0 or more, more preferably 3.2 or more, and further more preferably 3.8 or more from the viewpoint of foaming at the time of washing. The pH of the hair cleansing composition of the present invention at 25°C is, as the pH of a 5% water dispersion, preferably 7.0 or less, and more preferably 6.5 or less from the viewpoint of suppressing entanglement of hair and so on. In addition, the pH of the hair cleansing composition of the present invention at 25°C is, as the pH of a 5% water dispersion, preferably from 3.0 to 7.0, more preferably from 3.2 to 6.5, and further more preferably from 3.8 to 6.5.

The hair cleansing composition of the present invention usually contains an aqueous medium. Examples of the aqueous medium include water; lower alcohol, such as ethanol and isopropyl alcohol; and low molecular weight diol and triol having 6 or less carbon atoms, such as 1,3-butylene glycol, glycerin, ethylene glycol, and propylene glycol, and water is preferable. The aqueous medium means all aqueous media included in the hair cleansing composition including inevitably contained media such as water that are included, for example, the aqueous medium that is used when the component (B) is prepared by sulfonating a raw material olefin.

Although the content of the aqueous medium can be appropriately selected depending on the dosage form of the hair cleansing composition, the content in the hair cleansing composition is usually from 5 to 99 mass% and preferably from 30 to 98 mass%.

Thus, the hair cleansing composition of the present invention applied to hair expresses performances of bringing good foaming and also smooth foam quality at the time of washing and enabling to really feel softness and less friction feeling of hair at the time of rinsing, and the composition also has excellent low-temperature stability.

The objects to which the hair cleansing composition of the present invention is applied are hair, a scalp, and a head decoration product including a head decoration product fiber.

Here, the head decoration product fiber can be either a naturally derived fiber or a synthetic fiber and is preferably a naturally derived fiber.

The naturally derived fiber is a fiber harvested from a wild animal or plant (excluding human hair) or a fiber artificially manufactured using a protein, a polysaccharide, or the like as a raw material. Among these naturally derived fibers, preferred are animal hair and fibers artificially manufactured using, as raw materials, proteins, such as keratin, collagen, casein, and a proteins derived from soybeans, peanuts, corn, silk, or the like, and polysaccharides, more preferred are regenerated protein fibers of which the raw materials are keratin, collagen, casein, soybean protein, peanut protein, corn protein, silk protein (e.g., silk fibroin) and so on, further preferred are regenerated protein fibers such as a regenerated collagen fiber of which the raw material is collagen and a regenerated silk fiber of which the raw material is silk fibroin, and further preferred is a regenerated collagen fiber.

The regenerated collagen fiber can be manufactured by a known technology. The composition of the regenerated collagen fiber does not have to be 100% collagen and may contain a natural polymer, a synthetic polymer, an additive, or the like for quality improvement. Furthermore, the regenerated collagen fiber may be post-processed or post-treated. In general, the form of the regenerated collagen fiber is preferably a filament wound on a bobbin or taken out from a box. Alternatively, in a process of manufacturing a regenerated collagen fiber, a filament that puts out during a drying step can be directly used.

Examples of the synthetic fiber include a fiber including a synthetic resin as a main component. The synthetic resin is, from the viewpoint of ease of manufacturing of a synthetic fiber and a viewpoint of obtaining a texture similar to that of hair, preferably a thermoplastic resin and more preferably one or more selected from the group consisting of a polyester resin, a polyamide resin, a polyimide resin, a polyamide imide resin, a vinyl chloride resin, a polycarbonate resin, a polyphenylene sulfide resin, and a modacrylic resin (copolymer of acrylonitrile and vinyl chloride). The "main component" means a component whose content in the synthetic fiber is preferably 50 mass% or more, more preferably 60 mass% or more, further preferably 70 mass% or more, further more preferably 80 mass% or more, and further preferably 90 mass% or more and 100 mass% or less.

The synthetic fiber can contain, in addition to the above synthetic resin, various components such as a flame retardant, a flame retardant promoter, a light or heat stabilizer, a fluorescent agent, an antioxidant, an antistatic agent, and ultraviolet absorber within a range that does not impair the effects of the present invention.

Accordingly, the use of the hair cleansing composition of the present invention is highly useful as a method for washing hair, a scalp, or a head decoration product including a head decoration product fiber. The washing method is specifically, for example, a washing method including wetting hair, a scalp, or a head decoration product including a head decoration product fiber with water in advance, applying the hair cleansing composition of the present invention to the hair, scalp, or head decoration product including a head decoration product fiber, followed by washing, and then rinsing with water.

The use of the hair cleansing composition of the present invention is high in utility also as a low-temperature stabilization method for a hair cleansing composition.

Regarding the above-described embodiments, the present invention further discloses the following hair cleansing composition:
[1] A hair cleansing composition containing the following components (A) and (B):
   (A) a glycolipid type biosurfactant; and
   (B) an internal olefin sulfonic acid having 16 carbon atoms or a salt, prepared by sulfonation of a raw material olefin having 16 carbon atoms and having an average double bond position of 3.9-position or more and 4.4-position or less;
[2] The hair cleansing composition according to the above [1], wherein the component (A) includes one or more selected from the group consisting of a rhamnolipid, a sophorolipid, a trehalose lipid, and a mannosylalditol lipid;
[3] The hair cleansing composition according to the above [1], wherein the component (A) includes one or more selected from the group consisting of a rhamnolipid and a sophorolipid;
[4] The hair cleansing composition according to any one of the above [1] to [3], wherein the content of the rhamnolipid in the component (A) is 50 mass% or more;
[5] The hair cleansing composition according to any one of the above [1] to [3], wherein the content of the rhamnolipid in the component (A) is 90 mass% or more;
[6] The hair cleansing composition according to any one of the above [1] to [5], wherein the content of the component (A) is 0.05 mass% or more and 4.0 mass% or less;
[7] The hair cleansing composition according to any one of the above [1] to [5], wherein the content of the component (A) is 0.5 mass% or more and 2.0 mass% or less;
[8] The hair cleansing composition according to any one of the above [1] to [7], wherein the average double bond position in the raw material olefin having 16 carbon atoms forming the component (B) is 4.0-position or more and 4.3-position or less;
[9] The hair cleansing composition according to any one of the above [1] to [7], wherein the average double bond position of the raw material olefin having 16 carbon atoms that forms the component (B) is 4.0-position or more and 4.2-position or less;
[10] The hair cleansing composition according to any one of the above [1] to [9], wherein in the raw material olefin having 16 carbon atoms, the content of the raw material olefin having a double bond position at the 2-position in the raw material olefin having 16 carbon atoms is 10 mass% or more and 35 mass% or less;
[11] The hair cleansing composition according to any one of the above [1] to [9], wherein in the raw material olefin having 16 carbon atoms, the content of the raw material olefin having a double bond position at the 2-position in the raw material olefin having 16 carbon atoms is 20 mass% or more and 24 mass% or less;
[12] The hair cleansing composition according to any one of the above [1] to [11], wherein in the raw material olefin having 16 carbon atoms, the content of the raw material olefin having a double bond position at the 3-position in the raw material olefin having 16 carbon atoms is 10 mass% or more and 30 mass% or less;
[13] The hair cleansing composition according to any one of the above [1] to [11], wherein in the raw material olefin having 16 carbon atoms, the content of the raw material olefin having a double bond position at the 3-position in the raw material olefin having 16 carbon atoms is 16 mass% or more and 19 mass% or less;
[14] The hair cleansing composition according to any one of the above [1] to [13], wherein in the raw material olefin having 16 carbon atoms, the content of the raw material olefin having a double bond position at the 4-position in the raw material olefin having 16 carbon atoms is 10 mass% or more and 30 mass% or less;
[15] The hair cleansing composition according to any one of the above [1] to [13], wherein in the raw material olefin having 16 carbon atoms, the content of the raw material olefin having a double bond position at the 4-position in the raw material olefin having 16 carbon atoms is 17 mass% or more and 19 mass% or less;
[16] The hair cleansing composition according to any one of the above [1] to [15], wherein in the raw material olefin having 16 carbon atoms, the content of the raw material olefin having a double bond position at the 5-position in the raw material olefin having 16 carbon atoms is 5 mass% or more and 25 mass% or less;
[17] The hair cleansing composition according to any one of the above [1] to [15], wherein in the raw material olefin having 16 carbon atoms, the content of a raw material olefin having a double bond position of 5-position is 13 mass% or more and 15 mass% or less in the raw material olefin having 16 carbon atoms;
[18] The hair cleansing composition according to any one of the above [1] to [17], wherein in the raw material olefin having 16 carbon atoms, the content of the raw material olefin having a double bond position at the 6-position in the raw material olefin having 16 carbon atoms is 5 mass% or more and 20 mass% or less;
[19] The hair cleansing composition according to any one of the above [1] to [17], wherein in the raw material olefin having 16 carbon atoms, the content of the raw material olefin having a double bond position at the 6-position in the raw material olefin having 16 carbon atoms is 11 mass% or more and 13 mass% or less;
[20] The hair cleansing composition according to any one of the above [1] to [19], wherein in the raw material olefin having 16 carbon atoms, the total content of the raw material olefins having a double bond position at the 7-position or the 8-position in the raw material olefin having 16 carbon atoms is 5 mass% or more and 25 mass% or less;
[21] The hair cleansing composition according to any one of the above [1] to [19], wherein in the raw material olefin having 16 carbon atoms, the total content of the raw material olefins having a double bond position at the 7-position or the 8-position in the raw material olefin having 16 carbon atoms is 12 mass% or more and 16 mass% or less;
[22] The hair cleansing composition according to any one of the above [1] to [21], wherein in the internal olefin sulfonic acid having 16 carbon atoms or a salt thereof as the component (B), the content of the internal olefin sulfonic acid having a sulfonate group at the 1-position or more and the 4-position or less or a salt thereof in the component (B) is 40 mass% or more and 75 mass% or less;
[23] The hair cleansing composition according to any one of the above [1] to [21], wherein in the internal olefin sulfonic acid having 16 carbon atoms or a salt thereof as the component (B), the content of the internal olefin sulfonic acid having a sulfonate group at the 1-position or more and the 4-position or less or a salt thereof is 55 mass% or more and 68 mass% or less in the component (B);
[24] The hair cleansing composition according to any one of the above [1] to [23], wherein in the internal olefin sulfonic acid having 16 carbon atoms or a salt thereof as the component (B), the content of the internal olefin sulfonic acid having a sulfonate group at the 2-position or a salt thereof in the component (B) is 10 mass% or more and 35 mass% or less;
[25] The hair cleansing composition according to any one of the above [1] to [23], wherein in the internal olefin sulfonic acid having 16 carbon atoms or a salt thereof as the component (B), the content of the internal olefin sulfonic acid having a sulfonate group at the 2-position or a salt thereof is 17 mass% or more and 25 mass% or less in the component (B);
[26] The hair cleansing composition according to any one of the above [1] to [25], wherein in the internal olefin sulfonic acid having 16 carbon atoms or a salt thereof as the component (B), the content of the internal olefin sulfonic acid having a sulfonate group at the 3-position or a salt thereof is 5 mass% or more and 30 mass% or less in the component (B);
[27] The hair cleansing composition according to any one of the above [1] to [25], wherein in the internal olefin sulfonic acid having 16 carbon atoms or a salt thereof as the component (B), the content of the internal olefin sulfonic acid having a sulfonate group at the 3-position or a salt thereof is 15 mass% or more and 20 mass% or less in the component (B);
[28] The hair cleansing composition according to any one of the above [1] to [27], wherein in the internal olefin sulfonic acid having 16 carbon atoms or a salt thereof as the component (B), the content of the internal olefin sulfonic acid having a sulfonate group at the 4-position or a salt thereof in the component (B) is 15 mass% or more and 30 mass% or less;
[29] The hair cleansing composition according to any one of the above [1] to [27], wherein in the internal olefin sulfonic acid having 16 carbon atoms or a salt thereof as the component (B), the content of the internal olefin sulfonic acid having a sulfonate group at the 4-position or a salt thereof in the component (B) is 19 mass% or more and 23 mass% or less;
[30] The hair cleansing composition according to any one of the above [1] to [29], wherein in the internal olefin sulfonic acid having 16 carbon atoms or a salt thereof as the component (B), the mass ratio of the content of the hydroxy form (HAS) to the content of the olefin form (IOS), hydroxy form/olefin form, is from 50/50 to 100/0;
[31] The hair cleansing composition according to any one of the above [1] to [29], wherein in the internal olefin sulfonic acid having 16 carbon atoms or a salt thereof as the component (B), the mass ratio of the content of the hydroxy form (HAS) to the content of the olefin form (IOS), (hydroxy form/olefin form), is from 75/25 to 95/5;
[32] The hair cleansing composition according to any one of the above [1] to [31], wherein the content of the component (B) is 0.01 mass% or more and 30 mass% or less;
[33] The hair cleansing composition according to any one of the above [1] to [31], wherein the content of the component (B) is 2 mass% or more and 8 mass% or less;
[34] The hair cleansing composition according to any one of the above [1] to [33], wherein the mass ratio of the content of the component (A) to the content of the component (B), (A)/(B), is 0.005 or more and 0.60 or less;
[35] The hair cleansing composition according to any one of the above [1] to [33], wherein the mass ratio of the content of the component (A) to the content of the component (B), (A)/(B), is 0.05 or more and 0.30 or less;
[36] The hair cleansing composition according to any one of the above [1] to [35], wherein the mass ratio of the content of the component (A) to the total content of the component (A) and the component (B), (A)/{(A)+(B)}, is 0.001 or more and 0.5 or less;
[37] The hair cleansing composition according to any one of the above [1] to [35], wherein the mass ratio of the content of the component (A) to the total content of the component (A) and the component (B), (A)/{(A)+(B)}, is 0.05 or more and 0.25 or less;
[38] The hair cleansing composition according to any one of the above [1] to [37], containing an anionic surfactant (C) other than the component (B);
[39] The hair cleansing composition according to any one of the above [1] to [37], containing one or more selected from the group consisting of a sulfonate, an amino acid salt, a sulfosuccinate, a sulfate ester salt, and a carboxylate as the anionic surfactant (C) other than the component (B);
[40] The hair cleansing composition according to the above [38] or [39], wherein the content of the component (C) is 0.01 mass% or more and 30 mass% or less;
[41] The hair cleansing composition according to the above [38] or [39], wherein the content of the component (C) is 1.5 mass% or more and 8.0 mass% or less;
[42] The hair cleansing composition according to any one of the above [38] to [41], wherein the mass ratio of the content of the component (B) to the content of the component (C), (B)/(C), is 0.01 or more and 100 or less;
[43] The hair cleansing composition according to any one of the above [38] to [41], wherein the mass ratio of the content of the component (B) to the content of the component (C), (B)/(C), is 0.3 or more and 8 or less;
[44] The hair cleansing composition according to any one of the above [38] to [43], wherein when an internal olefin sulfonic acid or a salt thereof other than those having 16 carbon atoms is included as a component (C), the mass ratio of the content of the component (B) to the total amount of the internal olefin sulfonic acids or salts thereof in the hair cleansing composition of the present invention, (B)/(total amount of internal olefin sulfonic acids or salts thereof), is 0.2 or more;
[45] The hair cleansing composition according to any one of the above [38] to [43], wherein when the component (C) includes an internal olefin sulfonic acid of which the number of carbon atoms is other than 16 or a salt thereof, the mass ratio of the content of the component (B) to the total amount of internal olefin sulfonic acids or salts thereof in the hair cleansing composition of the present invention, (B)/(total amount of internal olefin sulfonic acids or salts thereof), is 0.95 or more;
[46] The hair cleansing composition according to any one of the above [1] to [45], wherein a 5% aqueous dispersion of the hair cleansing composition of the present invention has a pH of 3.0 or more and 7.0 or less at 25°C;
[47] The hair cleansing composition according to any one of the above [1] to [45], wherein a 5% aqueous dispersion of the hair cleansing composition of the present invention has a pH of 3.8 or more and 6.5 or less at 25°C;
[48] The hair cleansing composition according to any one of the above [1] to [47], wherein the hair cleansing composition of the present invention contains an aqueous medium, and the content of the aqueous medium is from 5 to 99 mass%;
[49] The hair cleansing composition according to any one of the above [1] to [47], wherein the hair cleansing composition of the present invention contains an aqueous medium, and the content of the aqueous medium is from 30 to 98 mass%;
[50] Use of a hair cleansing composition to hair, a scalp, or a head decoration product including a head decoration product fiber, the hair cleansing composition containing the following components (A) and (B):
   (A) a glycolipid type biosurfactant; and
   (B) an internal olefin sulfonic acid having 16 carbon atoms or a salt, prepared by sulfonation of a raw material olefin having 16 carbon atoms and having an average double bond position of 3.9-position or more and 4.4-position or less;
[51] A washing method including applying a hair cleansing composition to a scalp or a head decoration product including a head decoration product fiber, followed by washing, and then rinsing with water, the hair cleansing composition containing the following components (A) and (B):
   (A) a glycolipid type biosurfactant; and
   (B) an internal olefin sulfonic acid having 16 carbon atoms or a salt, prepared by sulfonation of a raw material olefin having 16 carbon atoms and having an average double bond position of 3.9-position or more and 4.4-position or less;
[52] A low-temperature stabilization method of a hair cleansing composition by a hair cleansing composition containing the following components (A) and (B):
   (A) a glycolipid type biosurfactant; and
   (B) an internal olefin sulfonic acid having 16 carbon atoms or a salt, prepared by sulfonation of a raw material olefin having 16 carbon atoms and having an average double bond position of 3.9-position or more and 4.4-position or less; and
[53] Use of a hair cleansing composition for obtaining low-temperature stability, the hair cleansing composition containing the following components (A) and (B):
   (A) a glycolipid type biosurfactant; and
   (B) an internal olefin sulfonic acid having 16 carbon atoms or a salt, prepared by sulfonation of a raw material olefin having 16 carbon atoms and having an average double bond position of 3.9-position or more and 4.4-position or less.

### Examples

The present invention will now be specifically described based on examples. Unless otherwise indicated in the table, the content of each component indicates mass%.

In addition, the measurement methods for various physical properties are as follows.

### [Measurement methods for various physical properties]

### (i) Method for measuring double bond position of raw material olefin

The double bond position of a raw material olefin was measured by gas chromatography (hereinafter, abbreviated as GC). Specifically, a dithiolated derivative of a raw material olefin was prepared by reaction with dimethyl disulfide, and then each component was separated by GC. As a result, the double bond positions of the raw material olefin were determined from the respective peak areas.

The apparatuses and analysis conditions used for measurement are as follows: GC apparatus (trade name: HP6890, manufactured by Hewlett-Packard Company); column (trade name: Ultra-Alloy-1HT capillary column 30 m × 250 µm × 0.15 µm, manufactured by Frontier Laboratories Ltd.); detector (hydrogen flame ionization detector (FID)); injection temperature: 300°C; detector temperature: 350°C; and He flow rate: 4.6 mL/min.

### (ii) Method for measuring content in accordance with sulfonate group bonding position of sodium internal olefin sulfonate

Regarding sodium internal olefin sulfonate with an attached sulfonate group, each sodium internal olefin sulfonate content in accordance with the sulfonate group bonding position was measured by high performance liquid chromatography/mass spectrometer (HPLC-MS). Specifically, hydroxy forms with a sulfonate group attached were separated by high performance liquid chromatography (HPLC) and were each applied to mass spectrometer (MS) for identification. As a result, each content was determined from the HPLC-MS peak areas.

The apparatuses and conditions used for measurement were as follows: HPLC apparatus "LD20ASXR" (manufactured by Shimadzu Corporation); column "ODS Hypersil (registered trademark)" (4.6 × 250 mm, particle size: 3 µm, manufactured by Thermo Fisher Scientific); sample preparation (1 000 times dilution with methanol); eluent A (10 mM ammonium acetate added water); eluent B (10 mM ammonium acetate added methacrylonitrile/water = 95/5 (v/v) solution); gradient (0 minutes (A/B = 60/40) → from 15.1 to 20 minutes (30/70) → from 20.1 to 30 minutes (60/40)); MS apparatus "LCMS-2020" (manufactured by Shimadzu Corporation); ESI detector (anion detection m/z: 321.10 ((A) component having 16 or 18 carbon atoms)); column temperature (40°C); flow rate (0.5 mL/min); and injection volume (5 µL).

### (iii) Method for measuring mass ratio of hydroxy form/olefin form

The mass ratio of hydroxy form/olefin form of sodium internal olefin sulfonate was measured by HPLC-MS. Specifically, the hydroxy form and the olefin form were separated by HPLC and were respectively applied to MS for identification. As a result, the rate of each of them was determined from the HPLC-MS peak areas.

The apparatuses and conditions used for the measurement were as follows: HPLC apparatus (trade name: Agilent Technology 1100, manufactured by Agilent Technologies, Inc.); column (trade name: L-column ODS 4.6 × 150 mm, manufactured by Chemicals Evaluation and Research Institute, Japan); sample preparation (1 000 times dilution with methanol); eluent A (10 mM ammonium acetate added water); eluent B (10 mM ammonium acetate added methanol); gradient (0 minutes (A/B = 30%/70%) - 10 minutes (30%/70%) → 55 minutes (0%/100%) → 65 minutes (0%/100%) → 66 minutes (30%/70%) → 75 minutes (30%/70%)); MS apparatus (trade name: Agilent Technology 1100 MS SL (G1946D)); and MS detection (anion detection m/z 60-1 600, UV 240 nm).

### (iv) Method for measuring content of raw material olefin

The content of unreacted raw material olefin in the sodium internal olefin sulfonate was measured by GC. Specifically, ether and petroleum ether were added to a sodium internal olefin sulfate aqueous solution, and extraction was then performed to obtain olefin in the petroleum ether phase. As a result, the raw material olefin was quantitatively measured from the GC peak area.

The apparatuses and analysis conditions used for the measurement were as follows: GC apparatus (trade name: Agilent Technology 6850, manufactured by Agilent Technologies, Inc.); column (trade name: Ultra-Alloy-1HT capillary column 15 m × 250 µm × 0.15 µm, manufactured by Frontier Laboratories Ltd.); detector (hydrogen flame ionization detector (FID)); injection temperature: 300°C; detector temperature: 350°C; and He flow rate: 3.8 mL/min.

### (v) Method for measuring content of inorganic compound

The contents of inorganic compounds were measured by potentiometric titration and neutralization titration. Specifically, the content of Na₂SO₄ was quantitatively measured by determining the sulfate radical (SO₄²⁻) by potentiometric titration. The content of NaOH was quantitatively measured by neutralization titration with dilute hydrochloric acid.

### [Manufacturing Example A1: manufacturing of sophorolipid]

A sophorolipid was manufactured in accordance with the following procedure 1.) to 4.).

### 1.) Plate culture

A Candida bombicola NBRC10243 strain as an inoculum was inoculated in an agar medium petri dish containing 1% glucose, 1% yeast extract, 1% tryptone, and 1.5% agar by one platinum loop and was cultured at 30°C for 2 days.

### 2.) Preculture

A culture solution (100 mL) containing 1% glucose, 1% yeast extract, and 1% tryptone was placed in a Sakaguchi flask and was sterilized at a high temperature of 121°C for 20 minutes. After cooling, one platinum loop was inoculated in the culture medium, followed by spinner culture for 2 days at 30°C and a rotation speed of 120 r/min.

### 3.) Main culture

Distilled water (10 kg) was added to rapeseed oil (10 kg), and Lipase AY 30SD (manufactured by Amano Enzyme Inc.) was added thereto to a concentration of 0.1%. The rapeseed oil diluted by the above-described method was stirred at 30°C for 24 hours and was then left to stand for 1 hour, and the separated upper phase was collected. Distilled water (10 kg) was added to the collected upper phase, and the mixture was stirred for dispersion and was then left to stand for 1 hour again while warming at 30°C. The separated upper phase was collected as rapeseed oil-derived fatty acid. A culture solution containing 5% rapeseed oil-derived fatty acid, 12.5% glucose, 2% yeast extract, and 0.1% urea was prepared in a 15-L measuring flask. The culture solution was sterilized in a 30-L jar fermenter, and the preculture solution (300 mL) was then inoculated therein, followed by spinner culture at 30°C, a rotation speed of 300 r/min, and an aeration rate of 7.5 L/min (ventilation volume of 0.5 vvm) for 96 hours from the start of culturing.

The aeration oxygen concentration was kept at 21% throughout the entire culture period.

### 4.) Harvest of sophorolipid

The resulting culture solution was left to stand, and the generated precipitate was harvested. Distilled water heated to 75°C was added to the harvested precipitate, the mixture was stirred for dispersion and was then left to stand for 30 minutes, and the precipitate was collected again. The above washing procedure was repeated three times to obtain a sophorolipid.

### [Manufacturing Example b1: manufacturing of raw material olefin b1 having 16 carbon atoms]

1-Hexadecanol (product name: KALCOL 6098, manufactured by Kao Corporation, 7 000 g (28.9 mol)) and γ-alumina (manufactured by STREM Chemicals, Inc., 350 g (5 mass% with respect to the raw material alcohol)) as a solid acid catalyst were placed in a flask equipped with a stirrer, and reaction was performed by stirring at 280°C for 8 hours while circulating nitrogen (7 000 mL/min) in the system. The alcohol conversion rate after the completion of the reaction was 100%. The obtained crude alkene internal olefin was transferred to a distillation flask and was distilled at from 136°C to 160°C/4.0 mmHg to obtain a raw material olefin b1 having 16 carbon atoms with an olefin purity of 100%.

### [Manufacturing Example b2: manufacturing of raw material olefin b2 having 16 carbon atoms]

A raw material olefin b2 having 16 carbon atoms was obtained with an olefin purity of 100% as in Manufacturing Example b1 except that the reaction time was changed to 7.5 hours.

### [Manufacturing Example b3: manufacturing of raw material olefin b3 having 16 carbon atoms]

A raw material olefin b3 having 16 carbon atoms was obtained with an olefin purity of 100% as in Manufacturing Example b1 except that the reaction time was changed to 8.5 hours.

### [Manufacturing Example b4: manufacturing of raw material olefin b4 having 16 carbon atoms]

A raw material olefin b4 having 16 carbon atoms was obtained with an olefin purity of 100% as in Manufacturing Example b1 except that the reaction time was changed to 6.5 hours.

### [Manufacturing Example b5: manufacturing of raw material olefin b5 having 16 carbon atoms]

A raw material olefin b5 having 16 carbon atoms was obtained with an olefin purity of 100% as in Manufacturing Example b1 except that the reaction time was changed to 9.5 hours.

### [Manufacturing Example B1: manufacturing of sodium internal olefin sulfonate B1 having 16 carbon atoms]

The raw material olefin a1 obtained in Manufacturing Example b1 was put in a thin film sulfonation reactor having an outer jacket, and sulfonation reaction was performed using a sulfur trioxide gas under the condition of allowing cooling water of 10°C to pass through the reactor outer jacket. The molar ratio of SO₃/internal olefin during the sulfonation reaction was set to 1.01. The obtained sulfonated product was mixed with an alkali aqueous solution prepared with sodium hydroxide (alkali agent) in an amount of 1.04 times mol per mol of the theoretical acid value, and neutralization was performed by a continuous method at 30°C for 1 hour. The obtained neutralized product was heated in an autoclave at 170°C for 1 hour for hydrolysis to obtain sodium internal olefin sulfonate B1 having 16 carbon atoms. The content of the raw material olefin contained in the obtained sodium internal olefin sulfonate B1 having 16 carbon atoms was 0.4 mass%, and the content of the inorganic compound was 0.39 mass%.

### [Manufacturing Example B2: manufacturing of sodium internal olefin sulfonate B2 having 16 carbon atoms]

Sodium internal olefin sulfonate B2 having 16 carbon atoms was obtained as in Manufacturing Example B1 except that the raw material olefin b2 obtained in Manufacturing Example b2 was used as the raw material olefin. The content of the raw material olefin contained in the obtained sodium internal olefin sulfonate B2 having 16 carbon atoms was 0.7 mass%, and the content of the inorganic compound was 0.49 mass%.

### [Manufacturing Example B3: manufacturing of sodium internal olefin sulfonate B3 having 16 carbon atoms]

Sodium internal olefin sulfonate B3 having 16 carbon atoms was obtained as in Manufacturing Example B1 except that the raw material olefin b3 obtained in Manufacturing Example b3 was used as the raw material olefin. The content of the raw material olefin contained in the obtained sodium internal olefin sulfonate B3 having 16 carbon atoms was 0.5 mass%, and the content of the inorganic compound was 0.54 mass%.

### [Manufacturing Example B4: manufacturing of sodium internal olefin sulfonate B4 having 16 carbon atoms]

Sodium internal olefin sulfonate B4 having 16 carbon atoms was obtained as in Manufacturing Example B1 except that the raw material olefin b4 obtained in Manufacturing Example b4 was used as the raw material olefin. The content of the raw material olefin contained in the obtained sodium internal olefin sulfonate B4 having 16 carbon atoms was 0.4 mass%, and the content of the inorganic compound was 0.41 mass%.

### [Manufacturing Example B5: manufacturing of sodium internal olefin sulfonate B5 having 16 carbon atoms]

Sodium internal olefin sulfonate B5 having 16 carbon atoms was obtained as in Manufacturing Example B1 except that the raw material olefin b5 obtained in Manufacturing Example b5 was used as the raw material olefin. The content of the raw material olefin contained in the obtained sodium internal olefin sulfonate B5 having 16 carbon atoms was 0.3 mass%, and the content of the inorganic compound was 0.43 mass%.

Each of the physical property values of the obtained raw material olefins b1 to b5 and sodium internal olefin sulfonates B1 to B5 are shown in Tables 1 to 2.

**[Table 1]**

| Raw material olefin | | b1 | b2 | b3 | b4 | b5 |
|---|---|---|---|---|---|---|
| Number of carbon atoms | | 16 | 16 | 16 | 16 | 16 |
| Double bond distribution in raw material olefin (mass%) | 1-position | 1.8 | 2.4 | 2.3 | 2.3 | 0.9 |
| | 2-position | 21.8 | 23.2 | 20.7 | 29.7 | 19.2 |
| | 3-position | 18.7 | 18.7 | 16.8 | 22.7 | 16.1 |
| | 4-position | 18.6 | 18.2 | 17.5 | 17.3 | 15.7 |
| | 5-position | 14.3 | 13.9 | 14.7 | 11.1 | 16.6 |
| | 6-position | 11.4 | 11.2 | 12.9 | 8.0 | 13.2 |
| | 7-position | 6.8 | 6.2 | 7.6 | 4.5 | 9.2 |
| | 8-position | 6.8 | 6.2 | 7.6 | 4.5 | 9.2 |
| | Total | 100.0 | 100.0 | 100.0 | 100.0 | 100.0 |
| Average double bond position (DBP) | | 4.17 | 4.08 | 4.28 | 3.70 | 4.50 |

**[Table 2]**

| Sodium internal olefin sulfonate | | B1 | B2 | B3 | B4 | B5 |
|---|---|---|---|---|---|---|
| Raw material olefin | | b1 | b2 | b3 | b4 | b5 |
| Distribution of sulfonate group (mass%) | 1-position | 2.0 | 1.2 | 1.6 | 1.6 | 0.8 |
| | 2-position | 24.8 | 18.7 | 17.5 | 18.4 | 13.9 |
| | 3-position | 19.1 | 16.1 | 15.7 | 15.2 | 12.1 |
| | 4-position | 22.0 | 19.9 | 20.3 | 19.3 | 18.1 |
| | 5- to 9-positions | 32.1 | 44.2 | 45.0 | 45.5 | 55.0 |
| | Total | 100 | 100 | 100 | 100 | 100 |
| Hydroxy form | | 83.9 | 84.2 | 83.8 | 84.0 | 84.5 |
| Olefin form | | 16.1 | 15.8 | 16.2 | 16.0 | 15.5 |

### [Examples 1 to 10 and Comparative Examples 1 to 5]

Hair cleansing compositions having the compositions shown in Tables 3 to 4 were prepared by a usual method. Specifically, a component (A), a component (B), an appropriate amount of water, and a component (C) as needed were placed in a beaker and were heated to from 60 to 80°C, mixed, and cooled to room temperature. Subsequently, water was replenished, and the pH was adjusted to 6.0 with a pH adjuster (succinic acid or disodium succinate aqueous solution).

In comparative Examples, hair cleansing compositions were prepared by the above method appropriately using a peptide type biosurfactant instead of the component (A) and appropriately using the obtained sodium internal olefin sulfonate A4 or A5 instead of the component (B).

Each evaluation was performed using the obtained hair cleansing compositions in accordance with the following methods.

The results are shown in Tables 3 to 4.

### <<Evaluation of hair washing effect>>

The components shown below were placed in a beaker and were heated to 80°C and then mixed, and after confirmation of uniform dissolution, cooled to obtain plain shampoo.

### (Composition of plain shampoo)

| (Component) | (mass%) |
|---|---|
| Na polyoxyethylene lauryl ether sulfate (42.0% as EMAL E-27C (manufactured by Kao Corporation, active component: 27 mass%)) | 11.3 |
| Coconut fatty acid N-methylethanolamide (AMINON C-11S (manufactured by Kao Corporation)) | 3.0 |
| Citric acid | 0.2 |
| Methylparaben | 0.3 |
| Purified water | balance |
| Total | 100.0 |

A Japanese untreated hair tress having a mass of 20 g and a length of 20 cm was washed with the plain shampoo to obtain a hair tress for evaluation of foaming. The obtained hair tress for evaluation was sufficiently wetted with warm water of from 35°C to 40°C, and 1 g of any of the hair cleansing compositions was then applied to the hair bundle, followed by washing for 1 minute. Each of the items of "good foaming" and "smooth foam quality" at the time of washing and "softness of hair" and "less friction feeling" at the time of rinsing were evaluated by scores by five special panelists in accordance with the following evaluation criteria, and the average values thereof were determined.

The average value "3" was used as a standard and used as an index for evaluation. When the average score of five panelists was 3 points or more, it was judged to be acceptable.

5: Very good,
4: Good,
3: Somewhat good,
2: Bad,
1: Very bad.

### <<Measurement of coacervation amount>>

The weight of an empty centrifuge tube was measured with a precision balance, and 0.5 g of any of the hair cleansing compositions was put in the tube and was diluted to 10 g with ion-exchanged water. Subsequently, after centrifugation at 3000 rpm for 90 minutes, the supernatant was discarded, it was dried under reduced pressure at 80°C overnight with the lid open, and the weight after the drying was measured. The weight of the centrifuge tube was subtracted from the obtained measured value, and the weight per 100 mL centrifuge tube was determined as the coacervation amount (g/100 mL).

### <<Evaluation of low-temperature stability>>

The obtained hair cleansing compositions were placed in respective screw bottles and were stored in a thermostatic chamber of -5°C. Subsequently, whether precipitate was formed over time was visually checked and evaluated in accordance with the following criteria:
a: Formation of precipitate was not observed even after the lapse of 6 months;
b: Formation of precipitate was observed during the lapse of 1 month or more and less than 6 months; and
c: Formation of precipitate was observed before the lapse of less than 1 month.

**[Table 3]**

| | | Example | Example | Example | Example | Example | Example | Comparative Example | Comparative Example |
|---|---|---|---|---|---|---|---|---|---|
| | | 1 | 2 | 3 | 4 | 5 | 6 | 1 | 2 |
| (A) | Rhamnolipid*¹ | 0.05 | 0.10 | 0.50 | 2.00 | 3.00 | 4.00 | 1.00 | |
| (B) | Sodium C16 internal olefin sulfonate B1 | 7.00 | 7.00 | 7.00 | 7.00 | 7.00 | 7.00 | | 7.00 |
| (C) | Sodium cocoyl methyl taurine*² | 3.00 | 3.00 | 3.00 | 3.00 | 3.00 | 3.00 | 3.00 | 3.00 |
| (D) | Lauramidopropyl hydroxysultaine*³ | 2.70 | 2.70 | 2.70 | 2.70 | 2.70 | 2.70 | 2.70 | 2.70 |
| | Lauramidopropyl betaine*⁴ | 0.30 | 0.30 | 0.30 | 0.30 | 0.30 | 0.30 | 0.30 | 0.30 |
| (E) | Polyoxyethylene (6) lauryl ether*⁵ | 1.00 | 1.00 | 1.00 | 1.00 | 1.00 | 1.00 | 1.00 | 1.00 |
| | Cocoyl N-methylethanolamide*⁶ | 1.20 | 1.20 | 1.20 | 1.20 | 1.20 | 1.20 | 1.20 | 1.20 |
| | Lauryl glucoside*⁷ | 1.70 | 1.70 | 1.70 | 1.70 | 1.70 | 1.70 | 1.70 | 1.70 |
| | 2-Ethylhexylglyceryl ether*⁸ | 0.11 | 0.11 | 0.11 | 0.11 | 0.11 | 0.11 | 0.11 | 0.11 |
| (F) | Cationated hydroxyethyl cellulose X*⁹ | 0.10 | 0.10 | 0.10 | 0.10 | 0.10 | 0.10 | 0.10 | 0.10 |
| | Cationated hydroxyethyl cellulose Y*¹⁰ | 0.35 | 0.35 | 0.35 | 0.35 | 0.35 | 0.35 | 0.35 | 0.35 |
| | Preservative | 0.10 | 0.10 | 0.10 | 0.10 | 0.10 | 0.10 | 0.10 | 0.10 |
| | Ethanol | 0.75 | 0.75 | 0.75 | 0.75 | 0.75 | 0.75 | 0.75 | 0.75 |
| | Purified water | Balance | Balance | Balance | Balance | Balance | Balance | Balance | Balance |
| | pH | 6.0 | 6.0 | 6.0 | 6.0 | 6.0 | 6.0 | 6.0 | 6.0 |
| | Total | 100.00 | 100.00 | 100.00 | 100.00 | 100.00 | 100.00 | 100.00 | 100.00 |
| | (A)/(B) | 0.007 | 0.014 | 0.07 | 0.29 | 0.43 | 0.57 | - | 0.00 |
| Foaming | | 4 | 5 | 5 | 5 | 5 | 5 | 2 | 3 |
| Smoothness of foam | | 4 | 5 | 5 | 5 | 4 | 4 | 1 | 3 |
| Softness of hair during rinsing | | 3 | 4 | 5 | 5 | 4 | 4 | 1 | 2 |
| Less friction feeling of hair during rinsing | | 3 | 4 | 5 | 5 | 5 | 2 | 2 | 1 |
| Coacervation amount [g/100 mL] | | 0.53 | 0.45 | 0.31 | 0.34 | 0.35 | 0.72 | 0.35 | 0.92 |
| Low-temperature stability | | a | a | a | a | b | b | a | a |

| | | | | | | | | | |
|---|---|---|---|---|---|---|---|---|---|
| *1: RHEANCE One, manufactured by Evonik Industries AG *2: DIAPON K-SF, manufactured by NOF Corporation *3: SOFTAZOLINE LSB-R, manufactured by Kawaken Fine Chemicals Co., Ltd. *4: SOFTAZOLINE LPB-R, manufactured by Kawaken Fine Chemicals Co., Ltd. *5: EMULGEN 108, manufactured by Kao Corporation *6: AMINON C-11S, manufactured by Kao Corporation *7: AG-124, manufactured by Kao Corporation *8: PENETOL GE-EH, manufactured by Kao Corporation *9: UCARE Polymer LR-400 (Polyquaternium-10), manufactured by The Dow Chemical Company *10: SoftCAT Polymer SL-30 (Polyquaternium-67), manufactured by The Dow Chemical Company | | | | | | | | | |

**[Table 4]**

| | | Example | Example | Example | Example | Example | Comparative Example | Comparative Example | Comparative Example |
|---|---|---|---|---|---|---|---|---|---|
| | | 4 | 7 | 8 | 9 | 10 | 3 | 4 | 5 |
| (A) | Rhamnolipid*¹ | 2.00 | | 2.00 | 2.00 | 2.00 | | 2.00 | 2.00 |
| | Sophorolipid*¹¹ | | 2.00 | | | | | | |
| | Peptide type biosurfactant*¹² | | | | | | 2.00 | | |
| (B) | Sodium C16 internal olefin sulfonate A1 (DBP4.17) | 7.00 | 7.00 | | | 7.00 | 7.00 | | |
| | Sodium C16 internal olefin sulfonate A2 (DBP4.08) | | | 7.00 | | | | | |
| | Sodium C16 internal olefin sulfonate A3 (DBP4.28) | | | | 7.00 | | | | |
| | Sodium C16 internal olefin sulfonate A4 (DBP3.70) | | | | | | | 7.00 | |
| | Sodium C16 internal olefin sulfonate A5 (DBP4.50) | | | | | | | | 7.00 |
| (C) | Sodium cocoyl methyl taurine*² | 3.00 | 3.00 | 3.00 | 3.00 | | 3.00 | 3.00 | 3.00 |
| (D) | Lauramidopropyl hydroxysultaine*³ | 2.70 | 2.70 | 2.70 | 2.70 | | 2.70 | 2.70 | 2.70 |
| | Lauramidopropyl betaine*⁴ | 0.30 | 0.30 | 0.30 | 0.30 | | 0.30 | 0.30 | 0.30 |
| (E) | Polyoxyethylene (6) lauryl ether*⁵ | 1.00 | 1.00 | 1.00 | 1.00 | | 1.00 | 1.00 | 1.00 |
| | Cocoyl N-methylethanolamide*⁶ | 1.20 | 1.20 | 1.20 | 1.20 | | 1.20 | 1.20 | 1.20 |
| | Lauryl glucoside*⁷ | 1.70 | 1.70 | 1.70 | 1.70 | | 1.70 | 1.70 | 1.70 |
| | 2-Ethylhexylglyceryl ether*⁸ | 0.11 | 0.11 | 0.11 | 0.11 | | 0.11 | 0.11 | 0.11 |
| (F) | Cationated hydroxyethyl cellulose X*⁹ | 0.10 | 0.10 | 0.10 | 0.10 | 0.10 | 0.10 | 0.10 | 0.10 |
| | Cationated hydroxyethyl cellulose Y*¹⁰ | 0.35 | 0.35 | 0.35 | 0.35 | 0.35 | 0.35 | 0.35 | 0.35 |
| | Preservative | 0.10 | 0.10 | 0.10 | 0.10 | 0.10 | 0.10 | 0.10 | 0.10 |
| | Ethanol | 0.75 | 0.75 | 0.75 | 0.75 | 0.75 | 0.75 | 0.75 | 0.75 |
| | Purified water | Balance | Balance | Balance | Balance | Balance | Balance | Balance | Balance |
| | pH | 6.00 | 6.00 | 6.00 | 6.00 | 6.00 | 6.00 | 6.00 | 6.00 |
| | Total | 100.00 | 100.00 | 100.00 | 100.00 | 100.00 | 100.00 | 100.00 | 100.00 |
| | (A)/(B) | 0.29 | 0.29 | 0.29 | 0.29 | 0.29 | 0.29 | 0.29 | 0.29 |
| Foaming | | 5 | 4 | 5 | 5 | 4 | 3 | 5 | 5 |
| Smoothness of foam | | 5 | 5 | 5 | 5 | 4 | 4 | 5 | 4 |
| Softness of hair during rinsing | | 5 | 5 | 5 | 4 | 4 | 1 | 5 | 2 |
| Less friction feeling of hair during rinsing | | 5 | 5 | 5 | 5 | 3 | 1 | 5 | 2 |
| Coacervation amount [g/100 mL] | | 0.34 | 0.36 | 0.32 | 0.31 | 0.26 | 0.19 | 0.39 | 0.66 |
| Low-temperature stability | | a | a | a | a | b | a | c | a |

| | | | | | | | | | |
|---|---|---|---|---|---|---|---|---|---|
| *1 to 10: Same as Table 3 *11: Sophorolipid obtained in Manufacturing Example A1 *12: KANEKA Surfactin, manufactured by KANEKA Corporation | | | | | | | | | |

## Claims

1. A hair cleansing composition comprising the following components (A) and (B):
(A) a glycolipid type biosurfactant; and
(B) an internal olefin sulfonic acid having 16 carbon atoms or a salt thereof, prepared by sulfonation of a raw material olefin having 16 carbon atoms and having an average double bond position of 3.9-position or more and 4.4-position or less.

2. The hair cleansing composition according to claim 1, wherein a mass ratio of a content of the component (A) to a content of the component (B), (A)/(B), is 0.005 or more and 0.60 or less.

3. The hair cleansing composition according to claim 1 or 2, wherein a content of the component (A) is 0.05 mass% or more and 4 mass% or less.

4. The hair cleansing composition according to any one of claims 1 to 3, wherein a content of an internal olefin sulfonic acid having a sulfonate group at the 1-position or more and the 4-position or less or a salt thereof is 40 mass% or more and 75 mass% or less in the component (B).

5. The hair cleansing composition according to any one of claims 1 to 4, wherein the component (A) includes one or more selected from the group consisting of a rhamnolipid, a sophorolipid, a trehalose lipid, and a mannosylalditol lipid.

6. The hair cleansing composition according to any one of claims 1 to 5, wherein a content of an internal olefin sulfonic acid having a sulfonate group at the 2-position or a salt thereof is 10 mass% or more and 35 mass% or less in the component (B).

7. The hair cleansing composition according to any one of claims 1 to 6, wherein a content of the component (B) is 0.01 mass% or more and 30 mass% or less.
